# EUROPEAN PATENT SPECIFICATION

(11) **EP 3 360 563 B1**
(45) Date of publication and mention of the grant of the patent: **03.04.2019**
(21) Application number: 17155660.8
(22) Date of filing: 10.02.2017
(51) Int. Cl.: A61P 19/00, A61K 35/64, A61K 36/21, A61K 36/23, A61K 36/238, A61K 36/25, A61K 36/254, A61K 36/46, A61K 36/65, A61K 36/11

(54) **THE PHARMACEUTICAL COMPOSITIONS FOR PREVENTION OR TREATMENT OF INFLAMMATORY SPINE DISEASE CONTAINING SCOLOPENDRA SUBSPINIPES AND PEONY AS AN ACTIVE INGREDIENT**
PHARMAZEUTISCHE ZUSAMMENSETZUNGEN ZUR VORBEUGUNG ODER BEHANDLUNG VON ENTZÜNDLICHEN WIRBELSÄULENERKRANKUNGEN MIT SCOLOPENDRA SUBSPINIPES UND PÄONIE ALS WIRKSTOFF
COMPOSITIONS PHARMACEUTIQUES POUR LA PRÉVENTION OU LE TRAITEMENT DE MALADIES INFLAMMATOIRES DE LA COLONNE VERTÉBRALE CONTENANT DES SCOLOPENDRA SUBSPINIPES ET DE LA PIVOINE COMME PRINCIPE ACTIF

(43) Date of publication of application: 15.08.2018
(73) Proprietor: Shin, Joon Shik, Seoul 00617 (KR)
(72) Inventor: SHIN, Joon Shik, 00617 Seoul (KR); LEE, Jin Ho, 06017 Seoul (KR)
(74) Representative: Pronovem

(56) References cited:
- US-B2- 8 293 289
- DATABASE TCM [Online] SIPO; 1 November 2011 (2011-11-01), Puer National Traditional MedicineResearch Institute; Zhou Bing: "A kind of Herba Spilanthis callimorphae and Radix Plumbaginis Zeylanicae tablet/A kind of new Chinese medicine composition for the treatment of prolapse of lumbar intervertebral disc and its preparation method", XP002770389, Database accession no. AN-201110172579-A & CN 102 228 594 A (PU ER NATION TRADITIONAL MEDICINE RES INST; BING ZHOU) 2 November 2011 (2011-11-02)
- Yang Jinlian ET AL: "Sixty-four cases of Scapulohumeral periarthritis treated by auricular plaster therapy", Journal of Traditional Chinese Medicine, vol. 26, no. 3 1 January 2006 (2006-01-01), pages 179-180, XP55374996, Retrieved from the Internet: URL:http://www.journaltcm.com/modules/Jour nal/contents/stories/063/8.pdf [retrieved on 2006-01-01]
- DATABASE WPI Week 200880 Thomson Scientific, London, GB; AN 2008-N70595 XP002770386, & CN 101 293 041 A (ZHANG J) 29 October 2008 (2008-10-29)
- DATABASE WPI Week 201623 Thomson Scientific, London, GB; AN 2016-08252V XP002770387, & CN 105 250 558 A (YAN Z) 20 January 2016 (2016-01-20)
- EUN-NI SEONG ET AL: "Study on the effect of aqua-acupuncture solution of Paeonia lactiflora on Collagen-Induced Arthritis of Mouse", THE KOREAN ACUPUNCTURE AND MOXIBUSTION SOCIETY, KOREAN INTELLECTUAL PROPERTY OFFICE, vol. 17, no. 1, 21 March 2000 (2000-03-21) , pages 221-250, XP053010245, ISSN: 1229-1137
- CHENGJIN LI ET AL: "Inhibitory Effects of Scolopendra Pharmacopuncture on the Development and Maintenance of Neuropathic Pain in Rats: Possible Involvement of Spinal Glial Cells", JOURNAL OF ACUPUNCTURE AND MERIDIAN STUDIES, vol. 8, no. 5, 1 October 2015 (2015-10-01) , pages 236-244, XP55374992, AMSTERDAM, NL ISSN: 2005-2901, DOI: 10.1016/j.jams.2015.01.001
- YOON HO-SUK ET AL: "Inhibitory Effect of a Decoction Combined with Ostericum koreanum Maxim. and Aralia continentalis Kitagawa on Collagen II-induced Arthritis Mice", KOREAN JOURNAL OF ORIENTAL MEDICAL PRESCRIPTION, KOREAN INTELLECTUAL PROPERTY OFFICE, KR, vol. 21, no. 1, 30 June 2013 (2013-06-30), pages 161-176, XP053029060, ISSN: 1229-1218, DOI: 10.14374/HFS.2013.21.1.161
- LI X ET AL: "TCM treatment for protrusion of lumbar intervertebral disc - A report of 100 cases", JOURNAL OF TRADITIONAL CHINESE MEDICINE 200609 CN, vol. 26, no. 3, September 2006 (2006-09), pages 186-188, XP55375016, ISSN: 0255-2922

## Description

### BACKGROUND OF THE INVENTION

### 1. Field of the Invention

The present invention relates to a pharmaceutical composition comprising of Peony and Scolopendra subspinipes as an active ingredient for the prevention or treatment of inflammatory spine disease and pharmacopuncture using the same ingredients.

### 2. Description of the Related Art

The types of bone disease in Korea are diverse and the number of bone disease patients is increasing every year. Bone disease mainly presents as inflammatory bone disease. A major inflammatory bone disease is lumbar herniated intervertebral disc, which is mainly divided into bulging disc, protruded disc, extruded disc, and sequestrated disc according to hernitation shape. With bulging disc, the annulus fibrosis is pushed out at least 3 mm out of the normal intervertebral disc range, but annulus fibrosis rupture is not observed. With protruded disc, the nucleus pulposus is pushed out through the inner fibers but the outer annulus fibrosis remains intact. With extruded disc, even the outer annulus fibrosis is violated and the nucleus pulposus extrudes through the whole layer of annulus fibrosis, but the nucleus pulposus remains connected to the central area. With sequestrated disc, the extruded nucleus pulposus is separated from the central area and has migrated into the spinal cavity. The characteristic symptoms such as lumbosacral radicular leg pain, sensory deficit, muscular weakness, and paralysis are presumed to be attributed to inflammatory response to chemical stimuli in the course of extrusion of nucleus pulposus or annulus fibrosis within the dura mater or autoimmune response rather than mechanical compression caused by the extruded disc itself.

Arthritis, another major inflammatory bone disease, is a skeletal inflammatory disease mainly caused by aging. Approximately 20% of the total population of Korea suffers from arthritis. There are almost 100 sub-types of arthritis, but osteoarthritis takes up the greater majority.

Osteoarthritis is a disease where cartilage in the joint is worn out so that articular bones collide against each other, causing pain. Muscles surrounding the joint are disused and accordingly lose motor power. Osteoarthritis is alternately known as degenerative joint disease, which is presumed to be caused by joint dysfunction or joint injury or even without joint injury.

Bone disease treatment methods such as chemotherapy and surgical operation still fail to achieve complete success. For example, while osteoarthritis has been treated using corticosteroids such as hydrocortisone and betamethasone which function by inhibiting prostaglandin synthesis, administration of such steroids is temporarily effective but needs to be carefully observed in the long term for serious side effects. Also, standard administration method is limited to intra-joint injection as opposed to oral administration.

Considering there is no satisfactory treatment method in spite of the increase in inflammatory bone disease patients, development of novel materials and novel treatment methods for clinical care of inflammatory bone disease is urgently needed.

Peony is a perennial root grass belonging to the *Ranunculaceae* family and its scientific name is *Paeonia lactiflora.* Peony is also known as peony flower. In Korea, Peony is the third most frequently used medicinal plant following ginseng and angelica, and is widely used as a raw material for decoctions needing preparation and medicinal herb products such as Ssanghwatang and Samultang. Peony root has generally been prescribed for the regulation of amenorrhea, the treatment of trauma, epistaxis, inflammation, and furuncle and wound healing, and the alleviation of chest and rib pain to treat inflammation, pain, and convulsion in Korean medicine. The bioactive constituents of Peony root are monoterpene glucoside compounds including paeoniflorin and albiflorin, and several pheonolic compounds whose chemical structures were previously disclosed (Kim, S. J., et al., Isolated and Identification of Biological Activity Compounds from Leaves and Stem of Paeonia lactiflora Pallas, Korean Journal of Medicinal Crop Science 15(1), 6-11, 2007).

*Scolopendra subspinipes* is the whole insect of *Scolopendra morsitans* L. and *Scolopendra subspinipes mitilans* L. which are arthropodas belonging to the *Scolopendridae* family. *Scolopendra subspinipes* is also called cheonryong, baekag, or centipede. Following collection in spring, the head, tail, and legs that have poison are removed from the body, and the body undergoes boiling in water. The boiled body is then dried under the sun, eaten directly without drying, pulverized into powder, or prepared spiritus vinosus. The taste is Spicy and the character is Warm but poisonous. In the Liver, it dispels Wind, alleviates convulsion, fights poison, disperses congelations and neutralizes snake poison. Owing to the Wind dispelling and convulsion alleviating effect, it is prescribed to treat such symptoms as cramps, rigidity, clamped jaws, and opisthotonos caused by acute or chronic convulsion. When it is clinically used, it is mixed with such medicinal materials as *Buthus martensi Batryticatus Bombyx,* and *Uncaria.* It has been used to treat various orthopedic and skin diseases and poisons, ulceration due to abscess, and poisonous snake bite owing to its excellent detoxication activity (Min Kyo Shin, Clinical Traditional Herbalogy, Younglim-sa, p665, 1991).

*Cibotii Rhizoma* belongs to the Dicksoniaceae family. Herein, Cibotium barometz J. Smith distributed in tropical regions is used. It is written in folk-medicine on the basis of Korean medicine literature that *Cibotii Rhizoma* is effective in strengthening bones. Active ingredients of *Cibotium barometz* are known to be onitin, onitin 4-O-β-D-allopyranoside, onitin 4-O-β-Dglucopyranoside, and pterosin R (4-deoxy, 4-chloro-onitin). Onitin has been confirmed to have the function of relaxing smooth muscles (Yang, Meei-Shieu, Studies on the Twian fork medicine VI. Studies on onitin. Planta Medica, p25, 1986).

*Ostericum Koreanum* is a perennial plant belonging to *Dicotyledoneae Umbelliflorae Apiaceae.* This plant is distributed in valleys of mountains in Korea (Gyeongbuk, Gangwon, Gyeonggi, Pyeongbuk, and Hamgyeong) and in the north-eastern areas of China. Major components of *Ostericum Koreanum* are Koreanin, Angelikoreanol, and iso-imperatorin. In folk-medicine, it has been used to relieve paralysis and pain in bones. The root of *Ostericum Koreanum* has been prescribed for the treatment of cold, headache, neuralgia, rheumatism, arthritis, and stroke in Korean medicine.

*Eucommia ulmoides* is a deciduous tree belonging to the *Eucommiaceae* family. Its origin is around Sichuan province, China. This plant is rare, and is characterized by consisting one species, one genus, and one family by itself, and is dioecious like ginkgo trees. The pharmaceutical effect of *Eucommia ulmoides* include lowering blood pressure, decreasing blood cholesterol, sedating, pain-killing, and promoting diuresis.

*Achyranthes* is a perennial plant or the root thereof belonging to the *Amaranthaceae* family. The name *Achyranthes* is named for the swollen nodes of the plant similar to cow's knees. The general effect of *Achyranthes* is promoting blood circulation and removing Blood stasis, so that it is effective in treating menstrual irregularity and post-partum pain. It is also known to be effective in strengthening such organs as the Liver and Kidney.

*Acanthopanax* which is also called Siberian ginseng is a medicinal plant containing large amounts of saponin, as ginseng does, whose scientific name is *Acanthopanax sessiliflorus.* In Korean medicine, this plant is considered not only to be helpful for improving Liver function, detoxication and immune function, but also for cleansing blood, strengthening bone and muscle, treating the 5 kinds of strain and the 7 types of emotional stresses, and increasing stamina.

*Saposhnikovia divaricata* is a plant that has a three-year life cycle and belongs to the *Dicotyledoneae Umbelliflorae Apiaceae* family. It is distributed in the meadows or rocky mountain regions of Korea, China (Northeastern area, Huabei), Mongolia, and Siberia. This plant is cultivated as a medicinal plant. The active ingredients of *Saposhnikovia divaricata* are coumarin compounds such as imperatorin, psoralen, and bergapten. The root is taken from plants with no flower stalk in spring and fall, which is then dried and used as medicinal plant material. In Korean medicine, this plant has been administered for sweating, reducing fever, relieving pain, and alleviating convulsion.

*Aralia contientalis* is a perennial plant belonging to the *Araliaceae* family, which is distributed mainly in mountain regions. In Korean medicine, the rhizome and root of *Aralia contientalis* are collected in fall, the outer skin is peeled, and contents are dried under the sun. The prepared medicinal plant has been used to treat migraines. The active ingredients of *Aralia contientalis* known so far are vitamin B12, folic acid, limonene, sabinene, α-pinene, γ-terpinene, myrcene, and humulene (α-caryophyllene). According to previous reports, *Aralia contientalis* has the effect of promoting diuresis, increasing vigor, lowering blood pressure, and treating convulsion and ulcers.

In the course of developing a novel composition for the treatment of inflammatory spine disease, the present inventors confirmed that the Peony and *Scolopendra subspinipes* mixed extract is effective in treating inflammatory spine disease, leading to the completion of the current invention.

### SUMMARY OF THE INVENTION

It is the objective of the present invention to provide a pharmaceutical composition comprising of Peony and *Scolopendra subspinipes* as active ingredients for the prevention or treatment of inflammatory spine disease.

To achieve the above objective, the present invention provides a pharmaceutical composition comprising of Peony, *Scolopendra subspinipes, Cibotium barometz, Ostericum Koreanum, Eucommia ulmoides, Achyranthes, Acanthopanax, Saposhnikovia divaricata, and Aralia continentalis* extract as an active ingredient for the prevention or treatment of inflammatory spine disease, wherein the weight ratio of *Peony, Scolopendra subspinipes, Cibotium barometz, Ostericum Koreanum, Eucommia ulmoides, Achyranthes, Acanthopanax, Saposhnikovia divaricata,* and *Aralia continentalis* is 2:1:1:1:1:1:1:1:1.

The present invention also provides a pharmacopuncture using the pharmaceutical composition for the prevention or treatment of inflammatory spine disease of the invention.

### ADVANTAGEOUS EFFECT

The present invention relates to a pharmaceutical composition for preventing or treating inflammatory spine disease comprising of Peony, *Scolopendra subspinipes, Cibotium barometz, Ostericum Koreanum, Eucommia ulmoides, Achyranthes, Acanthopanax, Saposhnikovia divaricata, and Aralia continentalis* extract as an active ingredient, wherein the weight ratio of *Peony, Scolopendra subspinipes, Cibotium barometz, Ostericum Koreanum, Eucommia ulmoides, Achyranthes, Acanthopanax, Saposhnikovia divaricata,* and *Aralia continentalis* is 2:1:1:1:1:1:1:1:1. More specifically, the present inventors have confirmed that the mixed extract of Peony and *Scolopendra subspinipes* was effective in inflammation response inhibition by inhibiting the generation of nitrogen oxide (NO) and cytokines dose-dependently and observed that ear edema in the ear edema-induced mouse model was alleviated by treatment of the mixed extract of Peony and *Scolopendra subspinipes* dose-dependently. Also, the mixed extract of Peony and *Scolopendra subspinipes* mixed with *Cibotium barometz, Ostericum Koreanum, Eucommia ulmoides, Achyranthes, Acanthopanax, Saposhnikovia divaricata,* and *Aralia contientalis* was confirmed to be most superior in inhibiting the inflammatory reaction. Pain relief and disc improvement was verified in administration of the said mixed extract to lumbar herniated intervertebral disc patients via pharmacopuncture. Therefore, it is suggested that the pharmaceutical composition comprising the extract of Peony and *Scolopendra subspinipes* of the present invention can be efficiently used for the prevention or treatment of inflammatory spine disease.

### BRIEF DESCRIPTION OF THE DRAWINGS

The preferred application and indications of the present invention are best understood with reference to the accompanying drawings, wherein:
Figure 1 is a diagram illustrating the preparation method of the mixed extract of the invention.

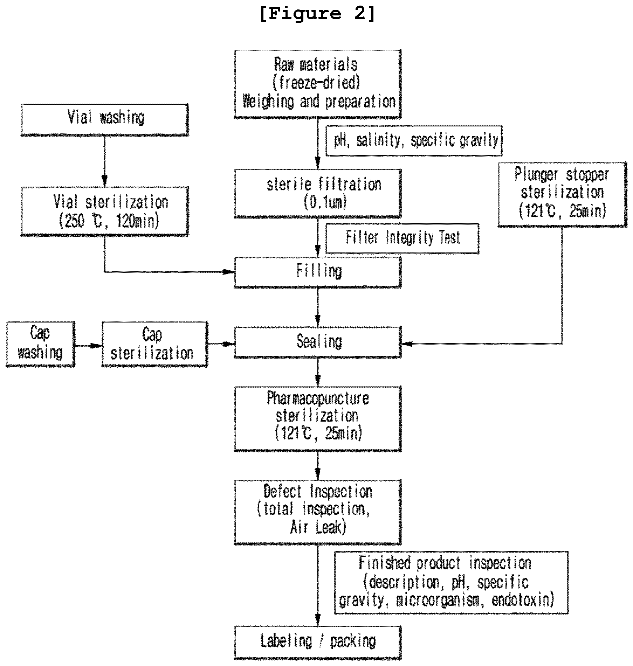

Figure 2 is a diagram illustrating the preparation method of pharmacopuncture using the mixed extract.
Figure 3 is a diagram illustrating the inhibitory effect of the mixed extract on the activity of nitrogen oxide (NO).
Figure 4 is a diagram illustrating the inhibitory effect of the mixed extract on cell proliferation.
Figure 5 is a diagram illustrating the inhibitory effect of the mixed extract on TNF-α, an inflammatory cytokine.
Figure 6 is a diagram illustrating the inhibitory effect of the mixed extract on IL-1β, an inflammatory cytokine.
Figure 7 is a diagram illustrating the inhibitory effect of the mixed extract on ear edema in ear edema-induced mouse.

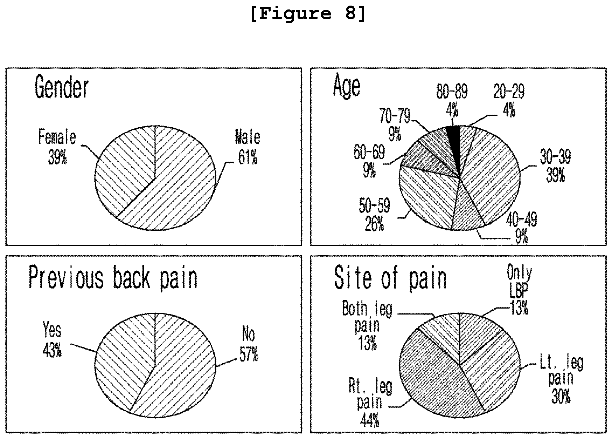

Figure 8 is a diagram illustrating the sex, age, and pathological characteristics of the lumbar herniated intervertebral disc patients who participated in this clinical study.
Figure 9 is a diagram illustrating the level of pain before and after administration of the mixed extract via pharmacopuncture, as evaluated using Numeric Rating Scale (NRS).
Figure 10 is a diagram illustrating the level of disc improvement in the lumbar herniated intervertebral disc patients after administration of the mixed extract via pharmacopuncture.

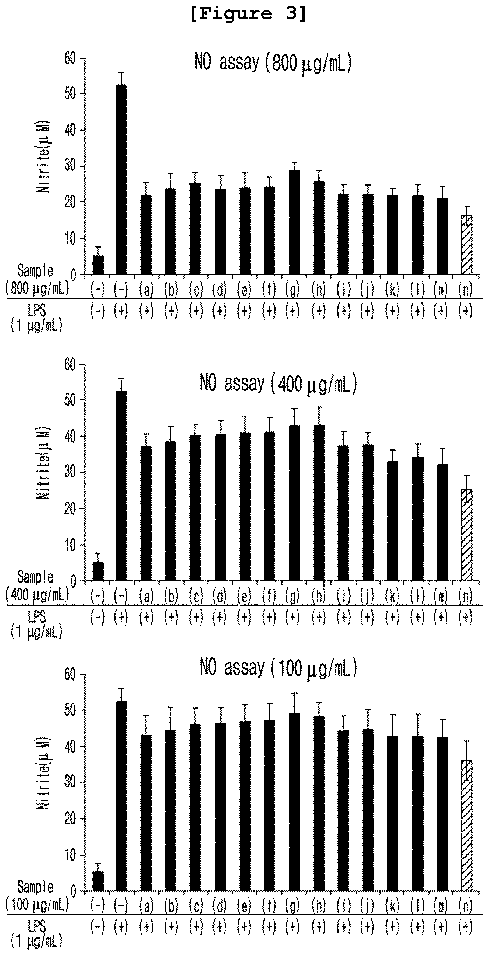

In Figures 3, 4, 5, 6, and 7, (a) indicates Peony and *Scolopendra subspinipes,* (b) indicates Peony, *Scolopendra subspinipes,* and *Cibotium barometz,* (c) indicates Peony, *Scolopendra subspinipes,* and *Ostericum Koreanum,* (d) indicates Peony, *Scolopendra subspinipes, Cibotium barometz,* and *Ostericum Koreanum,* (e) indicates Peony, *Scolopendra subspinipes, Cibotium barometz, Ostericum Koreanum,* and *Eucommia ulmoides,* (f) indicates Peony, *Scolopendra subspinipes, Cibotium barometz, Ostericum Koreanum,* and *Achyranthes,* (g) indicates Peony, *Scolopendra subspinipes, Cibotium barometz, Ostericum Koreanum,* and *Acanthopanax,* (h) indicates Peony, *Scolopendra subspinipes, Cibotium barometz, Ostericum Koreanum, Eucommia ulmoides,* and *Achyranthes,* (i) indicates Peony, *Scolopendra subspinipes, Cibotium barometz, Ostericum Koreanum, Eucommia ulmoides,* and *Acanthopanax,* (j) indicates Peony, *Scolopendra subspinipes, Cibotium barometz, Ostericum Koreanum, Achyranthes,* and *Acanthopanax,* (k) indicates Peony, *Scolopendra subspinipes, Cibotium barometz, Ostericum Koreanum, Eucommia ulmoides, Achyranthes,* and *Acanthopanax,* (1) indicates Peony, *Scolopendra subspinipes, Cibotium barometz, Ostericum Koreanum, Eucommia ulmoides, Achyranthes, Acanthopanax,* and *Saposhnikovia divaricata,* (m) indicates Peony, *Scolopendra subspinipes, Cibotium barometz, Ostericum Koreanum, Eucommia ulmoides, Achyranthes, Acanthopanax,* and *Aralia contientalis,* and (n) indicates Peony, *Scolopendra subspinipes, Cibotium barometz, Ostericum Koreanum, Eucommia ulmoides, Achyranthes, Acanthopanax, Saposhnikovia divaricata,* and *Aralia contientalis.*

### DESCRIPTION OF THE PREFERRED EMBODIMENT

Hereinafter, the present invention is described in detail.

The present invention provides a pharmaceutical composition comprising Peony, *Scolopendra subspinipes, Cibotium barometz, Ostericum Koreanum, Eucommia ulmoides, Achyranthes, Acanthopanax, Saposhnikovia divaricata, and Aralia continentalis* extract as an active ingredient for the prevention or treatment of inflammatory spine disease, wherein the weight ratio of *Peony, Scolopendra subspinipes, Cibotium barometz, Ostericum Koreanum, Eucommia ulmoides, Achyranthes, Acanthopanax, Saposhnikovia divaricata,* and *Aralia continentalis* is 2:1:1:1:1:1:1:1:1.

In this invention, the extract is prepared from the mixture of Peony, *Scolopendra subspinipes, Cibotium barometz, Ostericum Koreanum, Eucommia ulmoides, Achyranthes, Acanthopanax, Saposhnikovia divaricata,* and *Aralia contientalis,* wherein the weight ratio of *Peony, Scolopendra subspinipes, Cibotium barometz, Ostericum Koreanum, Eucommia ulmoides, Achyranthes, Acanthopanax, Saposhnikovia divaricata,* and *Aralia continentalis* is 2:1:1:1:1:1:1:1:1.

In this invention, the said Peony and *Scolopendra subspinipes* should be mixed at the ratio of 2:1. *Cibotium barometz, Ostericum Koreanum, Eucommia ulmoides, Achyranthes, Acanthopanax, Saposhnikovia divaricata,* and *Aralia contientalis* are added at the same dose as that of *Scolopendra subspinipes.*

In this invention, the extract can be extracted using water, C1 to C2 lower level alcohol, or a mixture thereof, and in the preferred embodiment example of the present invention, 70% ethanol was most preferred.

The possible extraction methods are shaking extraction, Soxhlet extraction, or reflux extraction, and the extraction solvent can be added at the volume of 1 to 40 times the volume of the dried Peony and *Scolopendra subspinipes.* The extraction temperature should be 20 to 50 °C, and the extraction time should be 10 to 100 hours. The extraction process may be repeated 1 to 5 times. The extract is then filtered and concentrated under reduced pressure using a vacuum concentrator or a vacuum rotary evaporator. Drying may be performed by reduced-pressure drying, vacuum drying, boiling drying, spray drying, or freeze drying.

In this invention, the inflammatory spine disease is preferably selected from within the group of lumbar herniated intervertebral disc, degenerative arthritis, and discitis.

In this invention, the composition can be a formulation for oral or parenteral administration, and most preferably a formulation for injection.

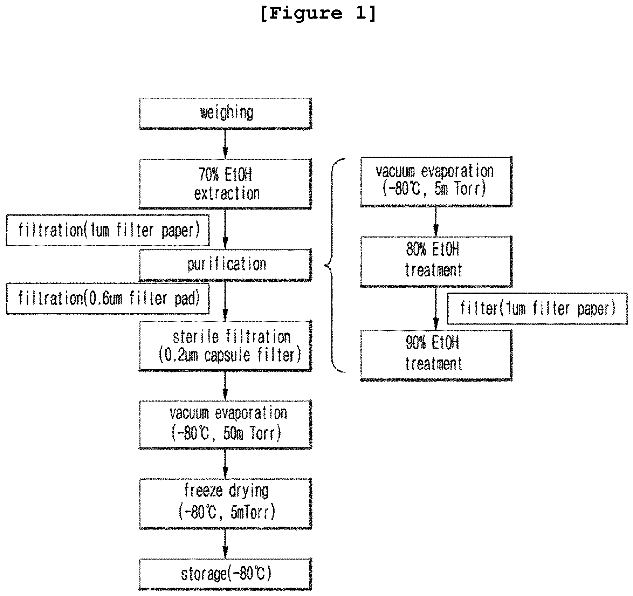

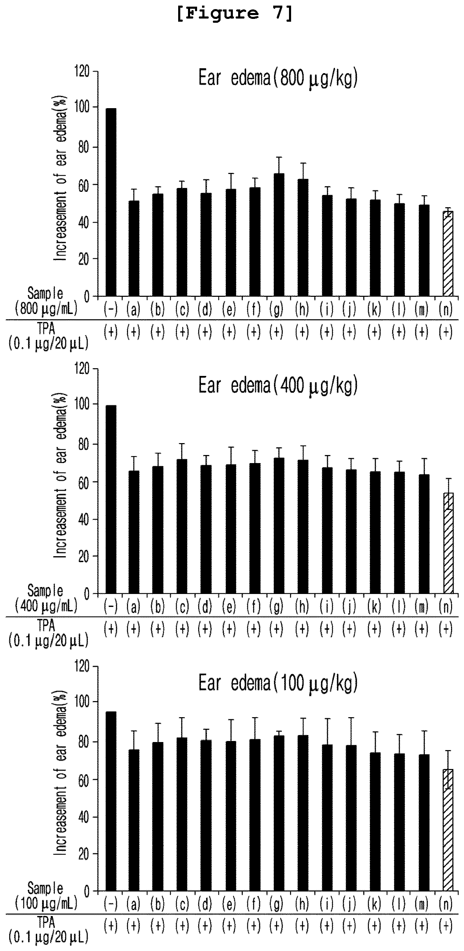

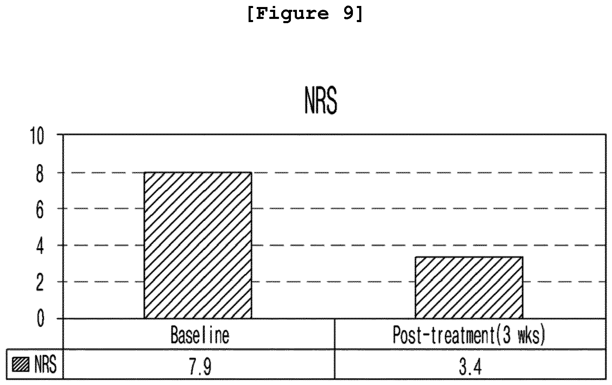

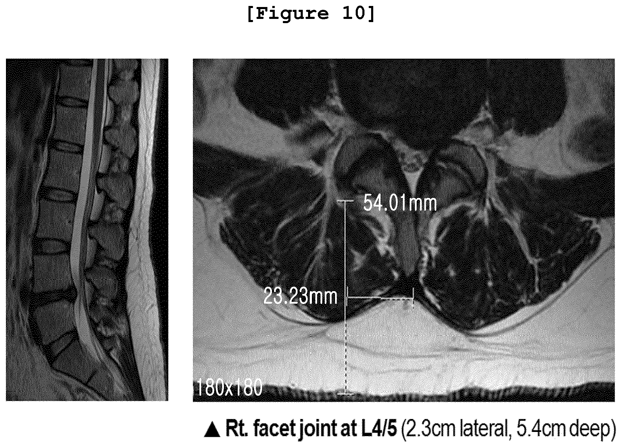

In an example, the inventors prepared the mixed extract of Peony and *Scolopendra subspinipes* (see Figure 1) and thereafter confirmed that the Peony and *Scolopendra subspinipes* mixed extract was effective in suppressing inflammatory response by inhibiting generation of NO and cytokines dose-dependently (see Figures 3, 5, and 6). In a mouse model induced with ear edema, the inventors also observed the alleviation of ear edema by treatment of the mixed extract, which was also dose-dependent (see Figure 7). In addition to the Peony and *Scolopendra subspinipes* mixed extract, the present inventors prepared the extract from the mixture of Peony, *Scolopendra subspinipes,* and *Cibotium barometz,* the extract from the mixture of Peony, *Scolopendra subspinipes,* and *Ostericum Koreanum,* the extract from the mixture of Peony, *Scolopendra subspinipes, Cibotium barometz,* and *Ostericum Koreanum,* the extract from the mixture of Peony, *Scolopendra subspinipes, Cibotium barometz, Ostericum Koreanum,* and *Eucommia ulmoides,* the extract from the mixture of Peony, *Scolopendra subspinipes, Cibotium barometz, Ostericum Koreanum,* and *Achyranthes,* the extract from the mixture of Peony, *Scolopendra subspinipes, Cibotium barometz, Ostericum Koreanum,* and *Acanthopanax,* the extract from the mixture of Peony, *Scolopendra subspinipes, Cibotium barometz, Ostericum Koreanum, Eucommia ulmoides,* and *Achyranthes,* the extract from the mixture of Peony, *Scolopendra subspinipes, Cibotium barometz, Ostericum Koreanum, Eucommia ulmoides,* and *Acanthopanax,* the extract from the mixture of Peony, *Scolopendra subspinipes, Cibotium barometz, Ostericum Koreanum, Achyranthes,* and *Acanthopanax,* the extract from the mixture of Peony, *Scolopendra subspinipes, Cibotium barometz, Ostericum Koreanum, Eucommia ulmoides, Achyranthes,* and *Acanthopanax,* the extract from the mixture of Peony, *Scolopendra subspinipes, Cibotium barometz, Ostericum Koreanum, Eucommia ulmoides, Achyranthes, Acanthopanax,* and *Saposhnikovia divaricata,* the extract from the mixture of Peony, *Scolopendra subspinipes, Cibotium barometz, Ostericum Koreanum, Eucommia ulmoides, Achyranthes, Acanthopanax,* and *Aralia contientalis,* and the extract from the mixture of Peony, *Scolopendra subspinipes, Cibotium barometz, Ostericum Koreanum, Eucommia ulmoides, Achyranthes, Acanthopanax, Saposhnikovia divaricata,* and *Aralia contientalis,* and confirmed the inhibitory effect of these extracts on inflammatory response. In particular, the present inventors observed that the extract prepared from the mixture of Peony, *Scolopendra subspinipes, Cibotium barometz, Ostericum Koreanum, Eucommia ulmoides, Achyranthes, Acanthopanax, Saposhnikovia divaricata,* and *Aralia contientalis* exhibited the strongest anti-inflammatory effect. Pain relief and disc improvement was observed upon administration of the extract prepared from the mixture of Peony, *Scolopendra subspinipes, Cibotium barometz, Ostericum Koreanum, Eucommia ulmoides, Achyranthes, Acanthopanax, Saposhnikovia divaricata,* and *Aralia contientalis* to lumbar herniated intervertebral disc patients (see Figures 9 and 10). Therefore, it is suggested that the pharmaceutical composition of the present invention comprising Peony and *Scolopendra subspinipes* extract can be effectively used for the prevention or treatment of inflammatory spine disease.

The composition of the present invention can additionally include a pharmaceutically acceptable carrier. The term "pharmaceutically acceptable" herein indicates that properties do not possess toxicity to cells or human beings exposed to the composition of the present invention. The composition containing the pharmaceutically acceptable carrier can be administered in various forms of oral or parenteral pharmaceutical formulation. That is, the composition of the present invention can be prepared for oral or parenteral administration by mixing with generally used diluents or excipients such as fillers, extenders, binders, wetting agents, disintegrating agents and surfactants. The above carriers, excipients and diluents may include one or more types of, but are not limited to lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, saline, methylhydroxybenzoate, propylhydroxybenzoate, talc, magnesium stearate, mineral oil, dextrin, calcium carbonate, propyleneglycol, and liquid paraffin, and all commonly used carriers, excipients and diluents may be used. All the above mentioned ingredients can be added to the active ingredient of Peony and *Scolopendra subspinipes* extract singly or together.

Solid formulations for oral administration include tablets, pills, powders, granules and capsules. These solid formulations are prepared by mixing one or more compounds with one or more excipients such as starch, calcium carbonate, sucrose or lactose, gelatin, etc. Also, in addition to simple excipients, such lubricants as magnesium stearate, and talc can be used. Liquid formulations for oral administrations include suspensions, solutions, emulsions and syrups, and the above-mentioned formulations can contain various excipients such as humectants, sweeteners, aromatics and preservatives in addition to generally used simple diluents such as water and liquid paraffin. Formulations for parenteral administration include sterilized aqueous solutions, water-insoluble excipients, suspensions, emulsions, lyophilized preparations, and suppositories. Water insoluble excipients and suspensions may include propylene glycol, polyethylene glycol, vegetable oils such as olive oil, and injectable esters such as ethylolate, etc. Suppositories may include witepsol, macrogol, tween 61, cacao butter, laurin butter, and glycerogelatin, etc.

The pharmaceutical composition of the present invention can be formulated in a form selected from the group of tablets, pills, powders, granules, capsules, solutions, emulsions, syrups, sterilized aqueous solutions, water-insoluble excipients, suspensions, emulsions, lyophilized preparations, and suppositories.

The pharmaceutical composition of the present invention may be administered at a pharmaceutically effective dose. The administration dose is not specifically limited and can be adjusted according to body absorption level, body weight, age, sex, and health condition of patient, diet, administration period, administration method, excretion rate, and severity of disease, etc. The pharmaceutical composition of the present invention should be prepared considering the effective dose range. The accordingly formulated unit dosage preparation can be administered under the supervision or observation of a specialist as needed or individual request several times at regular intervals using specialized administration methods. The composition of the present invention comprising the Peony and *Scolopendra subspinipes* extract can be preferably administered at doses of 0.5 to 5000 mg/kg per day, preferably at 50 to 500 mg/kg per day, and more preferably at 50 mg/kg per day. The frequency of the administration may be once a day or several times a day.

The present invention also provides pharmacopuncture comprising of the pharmaceutical composition of the invention for the prevention or treatment of inflammatory spine disease.

In this invention, the pharmaceutical composition for the prevention or treatment of inflammatory spine disease can be sterilized and/or additionally contain preservatives, stabilizers, wettable powders or emulsifiers, salts and/or buffers for the regulation of osmotic pressure, and other supplements or therapeutically useful materials, and the composition may be formulated using conventional methods.

The pharmaceutical composition for the prevention and treatment of inflammatory spine disease of the present invention can be formulated for pharmacopuncture use. The present invention further provides a pharmacopuncture composition for the prevention or treatment of inflammatory spine disease including the pharmaceutical composition of the invention for the prevention or treatment of inflammatory spine disease. Pharmacopuncture is a type of acupuncture where a purified medicine in liquid form is directly injected in certain areas such as meridian points or tender points. This acupuncture is prepared in injectable form, so that the medicinal extract prepared as an injectable solution can be injected at meridian points or the site of pain using syringes prepared in injection form. This is a new treatment method in Korean medicine that combines the therapeutic action of meridian acupoints and the pharmacological action of drugs. Korean medicine acupuncture generally involves acupoints and is divided into such acupuncture types as hand acupuncture, bloodletting acupuncture, Yeonja acupuncture, and other various execution types including those where the skin is needled directly or target area is stimulated deeply. Pharmacopuncture is
widely used as it stimulates meridian points which are the treatment target points in Korean medicine, and simultaneously produces skin injection results of herb medicinal extracts with unique pharmacological action. In addition, unlike conventional medicine injections where chemical drugs are injected into muscles or blood vessels, pharmacopuncture can be easily applied to emergency patients or patients with difficulty taking drugs orally.

Preferred implementation, preparation, and experiment examples are illustrated in detail below. Examples 1-13 are illustrative examples and example 14 is an example according to the present invention.

### Example 1: Preparation of the mixed extract of Peony and Scolopendra subspinipes

Peony and *Scolopendra subspinipes* were mixed at a ratio of 2:1, followed by extraction using 70% ethanol. The extract was then filtered with 1 *µ*m filter paper, followed by concentration under reduced pressure. The extract was treated with 80% ethanol and 90% ethanol, respectively, and then filtered and purified. The purified extract was filtered with 0.6 *µ*m filter paper, and filtered again using 0.2 *µ*m capsule filter to eliminate germs. After concentration under reduced pressure, the extract was freeze-dried at -80 °C, and then pulverized. The prepared extract was stored in frozen state.

### Example 2: Preparation of the mixed extract of Peony, Scolopendra subspinipes, and Cibotium barometz

Peony, *Scolopendra subspinipes,* and *Cibotium barometz* were weighed to a ratio of 2:1:1, and then extracted in the same manner as described in Example 1. The extract was pulverized and stored in frozen state.

### Example 3: Preparation of the mixed extract of Peony, Scolopendra subspinipes, and Ostericum Koreanum

Peony, Scolopendra subspinipes, and *Ostericum Koreanum* were weighed to a ratio of 2:1:1, and then extracted in the same manner as described in Example 1. The extract was pulverized and stored in frozen state.

### Example 4: Preparation of the mixed extract of Peony, Scolopendra subspinipes, Cibotium barometz, and Ostericum Koreanum

Peony, *Scolopendra subspinipes, Cibotium barometz,* and *Ostericum Koreanum* were weighed to a ratio of 2:1:1:1, and then extracted in the same manner as described in Example 1. The extract was pulverized and stored in frozen state.

### Example 5: Preparation of the mixed extract of Peony, Scolopendra subspinipes, Cibotium barometz, Ostericum Koreanum, and Eucommia ulmoides

Peony, *Scolopendra subspinipes, Cibotium barometz, Ostericum Koreanum,* and *Eucommia ulmoides* were weighed to a ratio of 2:1:1:1:1, and then extracted in the same manner as described in Example 1. The extract was pulverized and stored in frozen state.

### Example 6: Preparation of the mixed extract of Peony, Scolopendra subspinipes, Cibotium barometz, Ostericum Koreanum, and Achyranthes

Peony, *Scolopendra subspinipes, Cibotium barometz, Ostericum Koreanum,* and *Achyranthes* were weighed to a ratio of 2:1:1:1:1, and then extracted in the same manner as described in Example 1. The extract was pulverized and stored in frozen state.

### Example 7: Preparation of the mixed extract of Peony, Scolopendra subspinipes, Cibotium barometz, Ostericum Koreanum, and Acanthopanax

Peony, *Scolopendra subspinipes, Cibotium barometz, Ostericum Koreanum,* and *Acanthopanax* were weighed to a ratio of 2:1:1:1:1, and then extracted in the same manner as described in Example 1. The extract was pulverized and stored in frozen state.

### Example 8: Preparation of the mixed extract of Peony, Scolopendra subspinipes, Cibotium barometz, Ostericum Koreanum, Eucommia ulmoides, and Achyranthes

Peony, *Scolopendra subspinipes, Cibotium barometz, Ostericum Koreanum, Eucommia ulmoides,* and *Achyranthes* were weighed to a ratio of 2:1:1:1:1:1, and then extracted in the same manner as described in Example 1. The extract was pulverized and stored in frozen state.

### Example 9: Preparation of the mixed extract of Peony, Scolopendra subspinipes, Cibotium barometz, Ostericum Koreanum, Eucommia ulmoides, and Acanthopanax

Peony, *Scolopendra subspinipes, Cibotium barometz, Ostericum Koreanum, Eucommia ulmoides,* and *Acanthopanax* were weighed to a ratio of 2:1:1:1:1:1, and then extracted in the same manner as described in Example 1. The extract was pulverized and stored in frozen state.

### Example 10: Preparation of the mixed extract of Peony, Scolopendra subspinipes, Cibotium barometz, Ostericum Koreanum, Achyranthes, and Acanthopanax

Peony, *Scolopendra subspinipes, Cibotium barometz, Ostericum Koreanum, Achyranthes,* and *Acanthopanax* were weighed to a ratio of 2:1:1:1:1:1, and then extracted in the same manner as described in Example 1. The extract was pulverized and stored in frozen state.

### Example 11: Preparation of the mixed extract of Peony, Scolopendra subspinipes, Cibotium barometz, Ostericum Koreanum, Eucommia ulmoides, Achyranthes, and Acanthopanax

Peony, *Scolopendra subspinipes, Cibotium barometz, Ostericum Koreanum, Eucommia ulmoides, Achyranthes,* and *Acanthopanax* were weighed to a ratio of 2:1:1:1:1:1:1, and then extracted in the same manner as described in Example 1. The extract was pulverized and stored in frozen state.

### Example 12: Preparation of the mixed extract of Peony, Scolopendra subspinipes, Cibotium barometz, Ostericum Koreanum, Eucommia ulmoides, Achyranthes, Acanthopanax, and Saposhnikovia divaricata

Peony, *Scolopendra subspinipes, Cibotium barometz, Ostericum Koreanum, Eucommia ulmoides, Achyranthes, Acanthopanax,* and *Saposhnikovia divaricata* were weighed to a ratio of 2:1:1:1:1:1:1:1, and then extracted in the same manner as described in Example 1. The extract was pulverized and stored in frozen state.

### Example 13: Preparation of the mixed extract of Peony, Scolopendra subspinipes, Cibotium barometz, Ostericum Koreanum, Eucommia ulmoides, Achyranthes, Acanthopanax, and Aralia contientalis

Peony, *Scolopendra subspinipes, Cibotium barometz, Ostericum Koreanum, Eucommia ulmoides, Achyranthes, Acanthopanax,* and *Aralia contientalis* were weighed to a ratio of 2:1:1:1:1:1:1:1, and then extracted in the same manner as described in Example 1. The extract was pulverized and stored in frozen state.

### Example 14: Preparation of the mixed extract of Peony, Scolopendra subspinipes, Cibotium barometz, Ostericum Koreanum, Eucommia ulmoides, Achyranthes, Acanthopanax, Saposhnikovia divaricata, and Aralia contientalis

Peony, *Scolopendra subspinipes, Cibotium barometz, Ostericum Koreanum, Eucommia ulmoides, Achyranthes, Acanthopanax, Saposhnikovia divaricata,* and *Aralia contientalis* were weighed to a ratio of 2:1:1:1:1:1:1:1:1, and then extracted in the same manner as described in Example 1. The extract was pulverized and stored in frozen state.

### Manufacturing Example 1: Preparation of pharmacopuncture

To prepare pharmacopuncture using the extracts prepared in the abovementioned Examples 1 to 14, vials sterilized at 250 °C for 120 minutes were filled with the extracts sterilized by filtration, and sealed with sterilized caps. The prepared pharmacopuncture was sterilized at 121°C for 25 minutes. The sterilized pharmacopuncture was packaged as a final product after completing final product tests including investigation of impurities, properties, pH, specific gravity, and microorganisms.

### Example 15: Cell culture

To evaluate the immune associated activity of the mixed extract, the macrophage cell line Raw264.7 was cultured.

More specifically, Raw264.7 cells were obtained from American Type Culture Collection (ATCC), which were then cultured in Dulbecco's Modified Eagle Medium (DMEM, Gibco-Invitrogen) supplemented with 10% FBS (fetal bovine serum, Gibco-Invitrogen) and 1% penicillin-streptomycin (Gibco) under 37°C 5% CO₂ conditions. Sub-culture was performed every other day.

### Experimental Example 1: Confirmation of the inhibitory effect of the mixed extract on nitrogen oxide (NO) activity

The inhibitory effect of the mixed extracts prepared in Examples 1 to 14 on inflammatory response was investigated.

More specifically, the generation of nitrogen oxide (NO) was confirmed using the Raw264.7 cells cultured in Example 15. The cells were distributed in 96 well plates at a density of 2 × 10⁵ cells/1 mℓ, followed by culture in a 37°C 5% CO₂ incubator for 24 hours. The cultured cells were treated with the mixed extracts prepared in Example 1 to Example 14 at concentrations of 100, 400, and 800 *µ*g/mℓ, followed by culture for 6 hours. To induce inflammation, lipopolysaccharide (LPS) was added to each well of the plate at a concentration of 1 *µ*g/mℓ, and cultured for 18 hours. Supernatant was collected and treated with the same amount of Griess reagent. Then, NO generation was measured at 549 nm using a micro plate reader (TECAN Austria, Australia).

As a result, as shown in Figure 3, the generation of nitrogen oxide (NO) was reduced by the mixed extracts dose-dependently. In addition, NO generation decreased significantly in all the mixed extracts containing Peony and *Scolopendra subspinipes* compared with the control. In particular, the mixed extract prepared in Example 14 was confirmed to be superior in reducing NO generation (Figure 3).

### Experimental Example 2: Confirmation of the cytotoxicity of the mixed extract

Cytotoxicity of the mixed extracts prepared in Examples 1 to 14 of the present invention was investigated.

More specifically, the generation of nitrogen oxide (NO) was confirmed using the Raw264.7 cells cultured in Example 15. The cells were distributed in 96 well plates at a density of 2 × 10⁵ cells/1 mℓ, followed by culture in a 37°C 5% CO₂ incubator for 24 hours. The cultured cells were treated with the mixed extracts prepared in Example 1 to Example 14 at concentrations of 100, 400, and 800 *µ*g/mℓ, followed by culture for 6 hours. To induce inflammation, LPS was added to each well of the plate at a concentration of 1 *µ*g/mℓ, and cultured for 18 hours. Then, 20 *µ*ℓ of MTT reagent diluted in DPBS (10 mg/mℓ) was added to each well of the plate along with each test sample, followed by culture for 4 hours. After removing the supernatant, 50 *µ*ℓ of DMSO was added to each well of the plate, and shaken lightly. Optical density was measured at 570 nm using an ELISA reader (TECAN Austria, Australia) 30 minutes later. Cytotoxicity (%) was calculated by the ratio (%) of the optical density of the well treated with the extract to the optical density of the control well untreated with the extract.

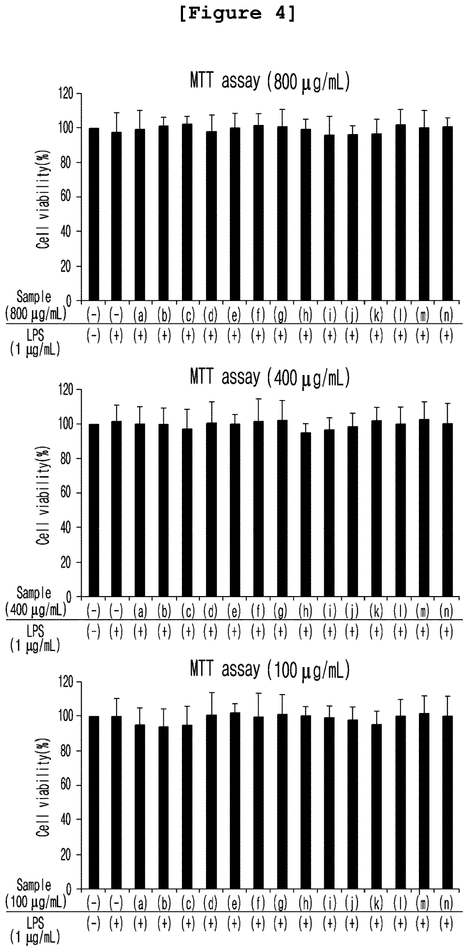

As a result, as shown in Figure 4, all the mixed extracts did not display cytotoxicity at any concentration (Figure 4).

### Experimental Example 3: Confirmation of the inhibitory effect of the mixed extract on inflammatory cytokine

The inhibitory effect of the mixed extracts prepared in Examples 1 to 14 on inflammatory cytokine was investigated.

More specifically, the Raw264.7 cells cultured in Example 15 were distributed in 96 well plates at a density of 1 × 10⁵ cells/mℓ, followed by culture for 24 hours. The cultured cells were treated with the mixed extracts prepared in Example 1 to Example 14 at concentrations of 100, 400, and 800 *µ*g/mℓ, and cultured for 24 hours. Supernatant was collected, followed by enzyme linked immumosorbent assay (ELISA) using an ELISA kit to measure cytokine concentration. A total 100 *µ*ℓ of the supernatant was added to each well of the plate coated with cytokine (TNF-α and IL-1β) antibodies (Abcam, England), and left to react at room temperature for 2 hours. Then, the supernatant was removed and the plate was washed with washing buffer comprising of PBS and Tween 20 at least 5 times. Detection antibody solution was added for antibody reaction, and Avidin-conjugated Horseradish Peroxidase (HRP) enzyme was added thereto, followed by reaction at room temperature for 15 minutes. Afterwards, stop solution (H₂SO₄) was added thereto to terminate the reaction between HRP enzyme and TMB matrix. Optical density was measured at 450 nm using a micro plate reader (TECAN Austria, Australia).

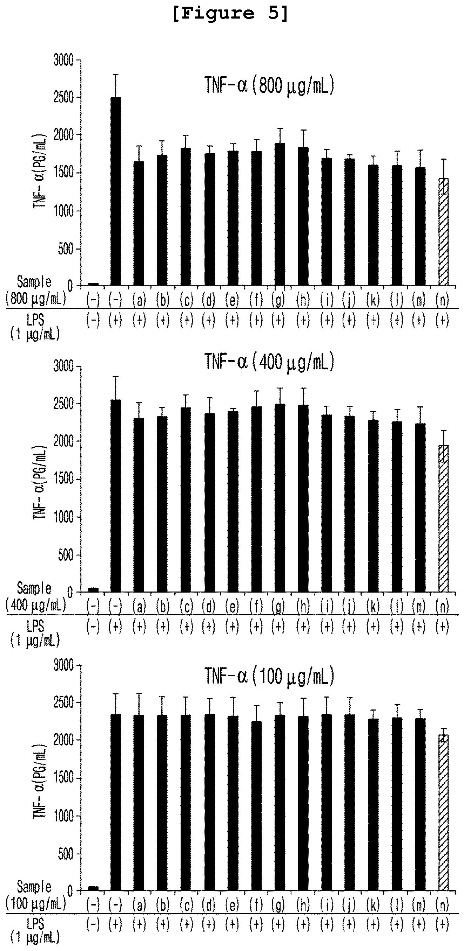

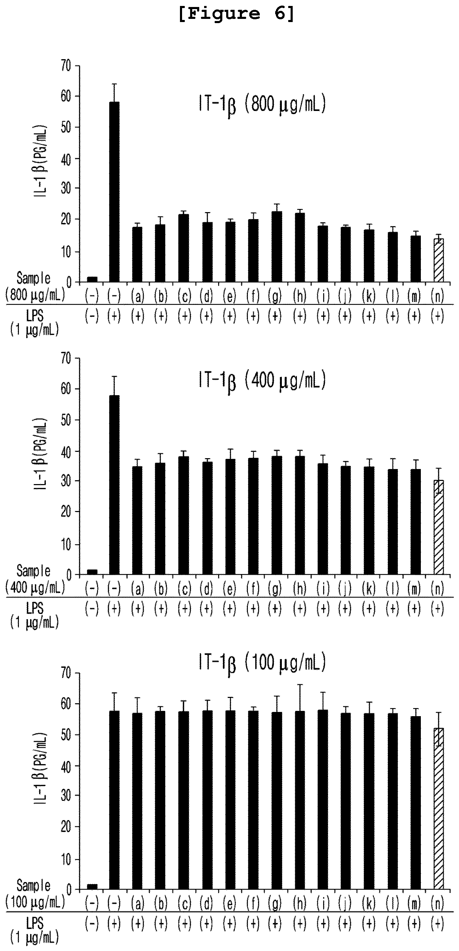

As a result, as shown in Figure 5 and Figure 6, the inhibitory effect on cytokine was confirmed in the group treated with the mixed extract dose-dependently. In particular, the mixed extract at the concentration of 800 *µ*g/mℓ displayed a considerable TNF-α and IL-1β inhibiting effect (Figure 5 and Figure 6). Among these mixed extracts, the mixed extract prepared in Example 14 showed the most substantial cytokine inhibiting effect (Figure 5 and Figure 6).

### Experimental Example 4: Confirmation of the inhibitory effect of the mixed extract on mouse ear edema

The inhibitory effect of the mixed extracts prepared in Examples 1 to 14 on mouse ear edema was investigated.

More specifically, 7 week old ICR mice were purchased from OrientBio (Korea). The test animals were distributed within each test group. The mice were treated with the mixed extracts prepared in Examples 1 to 14 at concentrations of 100, 400, and 800 *µ*g/kg via intramuscular injection, and 30 minutes later, 12-O-tetradecanoylphorbol-13-acetate (TPA) dissolved in acetone (1 *µ*g/20 *µ*ℓ) was administered to the mouse to induce edema in the ear. Only acetone was treated to the left ear as a control. To induce edema, the experimenter fixed the test animal tightly from the back and the second experimenter stimulated the edema inducing material using a micropipette. Both ears of the mouse were punched and weighed 4 hours later to measure edema severity.

As a result, as shown in Figure 7, ear edema was considerably improved by treatment of the mixed extract at the concentration of 800 *µ*g/kg. The alleviating effect on ear edema of the mixed extract was confirmed to be concentration dependent (Figure 7). Also, among the mixed extracts, the mixed extract prepared in Example 14 was the most effective in alleviating ear edema (Figure 7).

### Experimental Example 5: Confirmation of the improvement of lumbar herniated intervertebral disc by the mixed extract

The effect of the mixed extracts of the present invention on clinical patients was investigated.

More specifically, the inventors visited Jaseng Hospital of Korean Medicine, a spine specialty Hospital, in 2014. The mixed extract prepared in Example 14 was injected at acupuncture sites near the disc or hypertrophic joint using a 26 gauge syringe (4 cm) in 23 patients suffering from low back pain and lumbar radiculopathy diagnosed with lumbar herniated intervertebral disc. The level of pain and any adverse reactions were evaluated, and state was assessed by MRI.

The study participants were aged an average 49 ± 14 years, among which 14 (61%) patients were male, 10 patients (44%) had a history of low back pain, and 3 patients displayed muscular weakening or sensory loss. Three patients suffered from low back pain alone and the rest had lower extremity pain, among whom, 16 people (70%) had experienced pain radiating to below the knee. Currently, they had been treated clinically with conventional medicine treatment (14 patients were administered with pain killers, and 8 patients were treated with 1.9±1.5 nerve block sessions) and with Korean medicine, and were administered with the composition of the invention twice a week for 3 weeks. Evaluation of the level of pain was performed using Numeric Rating Scale (NRS) of lumbar disc herniation. The NRS measures the subjective intensity of low back pain, and consists of consecutive numbers from 0 to 10, in which 0 indicates no pain and 10 the most severe pain ever experienced. Patients were instructed to choose the number that best correlated with the pain at the time of measurement. The numbers were converted into scores.

As a result, as shown in Figure 9, the NRS significantly decreased from 7.9±1.7 to 3.4±1.6, and there were no reports of adverse reactions (Figure 9).

## Claims

1. A pharmaceutical composition for use in the prevention or treatment of inflammatory spine disease comprising extract of Peony, *Scolopendra subspinipes, Cibotium barometz, Ostericum Koreanum, Eucommia ulmoides, Achyranthes, Acanthopanax, Saposhnikovia divaricata,* and *Aralia continentalis* as an active ingredient,
wherein the weight ratio of Peony, *Scolopendra subspinipes, Cibotium barometz, Ostericum Koreanum, Eucommia ulmoides, Achyranthes, Acanthopanax, Saposhnikovia divaricata,* and *Aralia continentalis* is 2:1:1:1:1:1:1:1:1.

2. The pharmaceutical composition for use in the prevention or treatment of inflammatory spine disease according to Claim 1, wherein the extract is prepared by using water, C1 to C2 lower level alcohol, or a mixture thereof.

3. The pharmaceutical composition for use in the prevention or treatment of inflammatory spine disease according to Claim 1, wherein the inflammatory spine disease is selected from the group consisting of lumbar herniated intervertebral disc, degenerative arthritis, and discitis.

4. The pharmaceutical composition for use in the prevention or treatment of inflammatory spine disease according to Claim 1, wherein the composition is a formulation for injection.

5. A pharmacopuncture for use according to claim 1 including the pharmaceutical composition for the prevention or treatment of inflammatory spine disease of Claim 1.

## Patentansprüche

1. Pharmazeutische Zusammensetzung zur Verwendung bei der Prävention oder Behandlung einer entzündlichen Wirbelsäulenerkrankung, umfassend Extrakt von Pfingstrose, *Scolopendra subspinipes, Cibotium barometz, Ostericum Koreanum, Eucommia ulmoides, Achyranthes, Acanthopanax, Saposhnikovia divaricata* und *Aralia continentalis* als Wirkstoff,
wobei das Gewichtsverhältnis von Pfingstrose, *Scolopendra subspinipes, Cibotium barometz, Ostericum Koreanum, Eucommia ulmoides, Achyranthes, Acanthopanax, Saposhnikovia divaricata* und *Aralia continentalis* 2:1:1:1:1:1:1:1:1 beträgt.

2. Pharmazeutische Zusammensetzung zur Verwendung bei der Prävention oder Behandlung einer entzündlichen Wirbelsäulenerkrankung nach Anspruch 1, wobei der Extrakt unter Verwendung von Wasser, einem niederen C1- bis C2-Alkohol oder einer Mischung davon hergestellt wird.

3. Pharmazeutische Zusammensetzung zur Verwendung bei der Prävention oder Behandlung einer entzündlichen Wirbelsäulenerkrankung nach Anspruch 1, wobei die entzündliche Wirbelsäulenerkrankung aus der aus Lendenbandscheibenvorfall, degenerativer Arthritis und Diszitis bestehenden Gruppe ausgewählt ist.

4. Pharmazeutische Zusammensetzung zur Verwendung bei der Prävention oder Behandlung einer entzündlichen Wirbelsäulenerkrankung nach Anspruch 1, wobei es sich bei der Zusammensetzung um eine Injektionsformulierung handelt.

5. Pharmakopunktur zur Verwendung nach Anspruch 1, umfassend die pharmazeutische Zusammensetzung zur Prävention oder Behandlung einer entzündlichen Wirbelsäulenerkrankung nach Anspruch 1.

## Revendications

1. Composition pharmaceutique pour utilisation dans la prévention ou le traitement d'une maladie rachidienne inflammatoire comprenant un extrait de pivoine, *Scolopendra subspinipes, Cibotium barometz, Ostericum Koreanum, Eucommia ulmoides, Achyranthes, Acanthopanax, Saposhnikovia divaricata* et *Aralia continentalis* en tant que substance active,
dans laquelle le rapport en poids de pivoine, *Scolopendra subspinipes, Cibotium barometz, Ostericum Koreanum, Eucommia ulmoides, Achyranthes, Acanthopanax, Saposhnikovia divaricata* et *Aralia continentalis* est 2:1:1:1:1:1:1:1:1.

2. Composition pharmaceutique pour utilisation dans la prévention ou le traitement d'une maladie rachidienne inflammatoire selon la revendication 1, dans laquelle l'extrait est préparé en utilisant de l'eau, un alcool inférieur en C1 à C2, ou un mélange de ceux-ci.

3. Composition pharmaceutique pour utilisation dans la prévention ou le traitement d'une maladie rachidienne inflammatoire selon la revendication 1, la maladie rachidienne inflammatoire étant choisie dans le groupe constitué d'une hernie discale intervertébrale lombaire, d'une arthrite dégénérative et d'une discite.

4. Composition pharmaceutique pour utilisation dans la prévention ou le traitement d'une maladie rachidienne inflammatoire selon la revendication 1, la composition étant une formulation pour injection.

5. Pharmacopuncture pour utilisation selon la revendication 1 comprenant la composition pharmaceutique pour la prévention ou le traitement d'une maladie rachidienne inflammatoire selon la revendication 1.
